## Europäisches Patentamt
## European Patent Office
## Office européen des brevets

(11) Publication number: **0 067 812**
**B1**

(12)

# EUROPEAN PATENT SPECIFICATION

(45) Date of publication of patent specification: **28.08.85**

(21) Application number: **81901403.6**

(22) Date of filing: **30.12.80**

(86) International application number:
**PCT/US80/01747**

(87) International publication number:
**WO 82/02346 22.07.82 Gazette 82/18**

(51) Int. Cl.⁴: **B 01 D 53/34, C 01 B 17/02, C 01 B 17/20, C 01 G 49/02, C 09 K 7/00, C 09 K 3/00, C 22 B 7/02**

(54) **SCAVENGING HYDROGEN SULFIDE FROM HYDROCARBON GASES.**

(43) Date of publication of application:
**29.12.82 Bulletin 82/52**

(45) Publication of the grant of the patent:
**28.08.85 Bulletin 85/35**

(84) Designated Contracting States:
**DE FR GB NL SE**

(56) References cited:
**CA-A- 495 690
US-A-1 160 836
US-A-1 734 307
US-A-1 849 428
US-A-2 122 236
US-A-3 887 474
US-A-4 008 775
US-A-4 025 604
US-A-4 246 243
US-A-4 246 244**

**WORLD OIL, vol. 179, no. 1, July 1974, Gulf Publishing Co., HOUSTON, TEXAS (US), "New Mud Additive Will Neutralize H2S", pages 173-179**

(73) Proprietor: **GAS SWEETENER ASSOCIATES
7777 Bonhomme Avenue Suite 1402
Clayton, MO 63105 (US)**

(72) Inventor: **FOX, Irwin
37 Meadowbrook Country Club Estates
Ballwin St. Louis, MO 63011 (US)**

(74) Representative: **Hansen, Bernd, Dr.rer.nat. et al
Hoffmann, Eitle & Partner Patentanwälte
Arabellastrasse 4
D-8000 München 81 (DE)**

(56) References cited:
**Milbourne, "The removal of hydrogen sulfide from gases by means of iron oxide with special reference to humidity conditions", John Hopkins University, 1930, pages 9-11**

Courier Press, Leamington Spa, England.

**Description**

This invention relates to scavenging hydrogen sulfide from hydrocarbon gases such as natural gas.

Background art:

Hydrogen sulfide has been removed previously from hydrocarbon gases utilizing iron oxides. U.S. Patent No. 1,160,375 (Burkheiser) teaches that $H_2S$ may be removed from hydrocarbon gases containing ammonia by reaction with $Fe_2O_3$ and free sulfur. The reaction product, $Fe_2S_3$, is unstable in air, regenerating the $Fe_2O_3$ and liberating $H_2S$ which is reacted with ammonia in a second step to produce forms of ammonium sulfide. U.S. Patent No. 1,734,307 (Sperr) discloses a method of removing hydrogen sulfide by first reacting the $H_2S$ with an alkali such as sodium carbonate, and further reacting the resulting hydrogen sulfide salt with $Fe_2O_3$ to form $Fe_2S_3$. Canadian Patent No. 495,690 (Meuser) teaches that an $H_2S$-reactive iron oxide is produced during iron-mediated nitroaromatic reductions in the presence of both sulfuric acid and aluminum chloride. The iron oxide so produced is composed entirely of $Fe_2O_3$ and in reaction with hydrogen sulfide, forms an unstable iron sulfide which regenerates $H_2S$ upon acidification.

The foregoing methods appear to be effective only where the concentration of hydrogen sulfide is low, that is not in excess of about 15 ppm. Further, all of these iron oxides in reaction with $H_2S$ produce unstable products upon exposure to air or acids.

The principal object of the present invention is to provide a process for scavenging hydrogen sulfide from hydrocarbon gas, regardless of its concentration. A further purpose is to produce, in the reaction with hydrogen sulfide, reaction products which do not readily regenerate hydrogen sulfide gas in the presence of air or dissolved acids. A still further object is to provide a process in which $H_2S$ may be scavenged from hydrocarbon gas over a broad acidic-alkaline pH range and from any liquid in which the defined iron oxide particles may be suspended.

The present invention provides a process of scavenging hydrogen sulfide from hydrocarbon gas, comprising the steps of bringing such hydrocarbon gas into contact with iron oxide particles, which is characterized in that the iron oxide particles have a crystalline phase portion selected from the group consisting of $Fe_2O_3$, $Fe_3O_4$ and combinations thereof, together with an amorphous $Fe_2O_3$ portion and having a surface area of at least 4 $m^2/g$, and in that the said iron oxide particles are suspended in a liquid slurry, and in that the hydrocarbon gas containing such hydrogen sulfide to be scavenged is bubbled through said slurry, thereby aiding its circulation and thus maintaining said iron oxide particles in suspension, the hydrocarbon gas then escaping from said slurry being collected by known method.

Preferably said particles have impurity levels of any free element in bulk of less than 10.0% by weight and an $Fe^{+++}$ content intermediate to that for pure standards of $Fe_2O_3$ and $Fe_3O_4$, and preferably said particles comprise a crystalline $Fe_3O_4$ core surrounded by an amorphous $Fe_2O_3$ layer.

Simple apparatus serves to carry out the process, and includes conventional scrubbing columns, bubble chambers, and mixers such as static mixers. While the efficacy of $H_2S$-scavenging will vary for different types of apparatus, generally any such apparatus will serve which brings a gaseous hydrocarbon phase containing $H_2S$ into intimate mixing contact with the liquid-suspended particles. The simplicity of the apparatus is particularly advantageous in that it permits small inexpensive installations close to single gas wells.

The amount of iron oxide particles required for effective $H_2S$ removal will vary with $H_2S$ concentration and the degree of liquid-gas mixing. For hydrocarbon gases containing high $H_2S$ residuals and for apparatus wherein liquid-gas mixing is relatively inefficient (as in a conventional scrubbing column), the iron oxide content of slurries may be conveniently increased to provide an amount sufficient for effective scavenging. Alternatively, at low $H_2S$ residuals, iron oxides having lower rates of reaction, but which fix the sulfur under highly acidic conditions, may be preferable.

Best mode for carrying out the invention:

In the method of the present invention, iron oxide particles having unique physical and chemical properties are utilized in scavenging hydrogen sulfide from hydrocarbon gases. In contrast to those gas sweetening processes in which the gas passes through only slightly moistened beds of reactive material, and to those processes in which liquid bearing such material is sprayed downward within packed towers, in the present process the hydrocarbon gas polluted by hydrogen sulfide is bubbled through a liquid slurry of reactive particles under conditions hereafter more fully described which allow for full reaction; the hydrogen sulfide is reacted in the liquid slurry to a point that it is either completely eliminated or reduced to within the permissible limit of 4 ppm; and the purified gas is gathered from the upper surface of the liquid slurry.

The reactive particles utilized in the present process may be derived from several sources and may be prepared under various conditions. One such particle (hereinafter referred to as compound A) is described in U.S. Patent No. 4,008,775, and is prepared conventionally by controlled low temperature oxidation of iron. Other particles are obtained as the iron oxide waste dusts from steelmaking. These may be open hearth dust (hereinafter referred to as compound B) or basic oxygen furnace dust (hereinafter referred to as compound C) and are formed upon rapid solidification of iron droplets or vapor, as upon contact with furnace flue surfaces. Yet another useful particle (hereinafter referred to as compound D) is formed upon conventional high temperature oxidation of ferrous sulfate with rapid cooling. In general, I have found that any iron oxide will be effective in the defined process, regardless of source or method of manufacture, provided that it has the physical and chemical properties as hereinafter more fully described.

Table I lists representative iron oxides of the present invention (compounds A—D) together with certain control and prior art iron oxides, and summarizes those physical and chemical properties, hereafter explained, which are found to be significant.

TABLE I

| Iron oxide | Fe ESCA/ AUGER | X-ray Diffraction | | Kinetic "K" value | Acid stability | Surface area $m^2/g$ |
| | | Unreacted | Reacted | | | |
|---|---|---|---|---|---|---|
| A | $Fe_2O_3$ | $Fe_3O_4$ | $FeS_2$, $Fe_3S_4$, $FeS°$ | 702 | 86.6% | 7.0 |
| B | $Fe_2O_3$ | $Fe_2O_3/$ $Fe_3O_4$ | $FeS_2,S°$ | 12.7 | 73.7% | 9.0 |
| C | N.A. | $Fe_2O_3/$ $Fe_3O_4$ | $FeS_2,S°$ | 35.1 | 93.8% | 4.0 |
| D | $Fe_2O_3$ | $Fe_2O_3$ | $FeS_2$ | 1404 | 85.0% | 4.0 |
| O*[2] | $Fe_3O_4$ | $Fe_3O_4$ | N.A. | <0.35 | 18.7% | 1.0 |
| P[3] | N.A. | $Fe_3O_4$ | FeSx | <0.35 | 20.0% | 1.0 |
| Q[4] | $Fe_2O_3$ | $Fe_2O_3$ | FeS, $Fe_2Sx$ | <0.42 | 18.6% | 14.0 |
| R[5] | N.A. | N.A. | N.A. | <0.35 | 50.0% | N.A. |

N.A.=not analyzed
[2]=magnetite
[3]=hematite
[4]=iron oxide made according to the method of Meuser, Canadian Pat. No. 495,690
[5]=millscale

Samples of various effective particles were subjected to ESCA and AUGER analysis. In ESCA spectroscopy, a beam of monochromatic X-rays impinge upon the atoms at a sample surface, striking electrons and displacing them at energy levels characteristic for each valance state of element present. AUGER spectroscopy is similar to ESCA, except that the sample is bombarded with high-energy electrons instead of X-rays. AUGER electrons are emitted at characteristic energies for the various elements in particular valence states. These techniques analyze only the sample surface (less than 100 Å interior to the sample) in contrast to X-ray fluorescence which quantifies elements detected in bulk.

X-ray diffraction analysis detects only the crystalline phase of iron oxides; it will not yield a diffraction pattern for any iron oxide in which the Fe and O atoms are not organized into a crystalline lattice structure. It is concluded, from comparison of the ESCA/AUGER analysis and X-ray diffraction, that the $Fe_2O_3$ detected at the surface of any particle must be at least partially non-crystalline (amorphous).

Referring to Table I, ESCA/AUGER analysis of the present compound classes A, C and D shows that the surface of the particles comprises exclusively $Fe_2O_3$, regardless of crystalline content revealed by X-ray diffraction, which may be $Fe_3O_4$, $Fe_2O_3$ and combinations thereof. In the case of compound A, all of the $Fe_2O_3$ present must be amorphous since the only crystalline phase detected by X-ray diffraction analysis is $Fe_3O_4$. The amorphous nature of the $Fe_2O_3$ in compound D cannot be directly determined since all of the iron is present in its highest oxidation state as $Fe^{+++}$; however, the amorphous property may be inferred from the high kinetic K value of reaction with hydrogen sulfide; also, compound D appears to have a relatively low density compared to that of crystalline hematite (data not shown).

The amorphous $Fe_2O_3$ moiety of compound classes A, B and C was further identified by ion titration analysis. In ion titration, the amounts of iron in the $Fe^{++}$ and $Fe^{+++}$ oxidation states are measured simultaneously and compared to corresponding values for pure $Fe_2O_3$ and $Fe_3O_4$ standards. If the particles were composed substantially of $Fe_3O_4$, as indicated by strong X-ray diffraction patterns, then the ferric (Fe$^{+++}$) content would approximate 47.7% of the total iron, and Fe$^{++}$ iron would be present at approximately 27.6%.

Ion titration performed on the present iron oxide powders (compound classes, A, B and C) shows that Fe$^{+++}$ content is much higher than would be predicted by X-ray diffraction analysis; Fe$^{++}$ levels are correspondingly lower than for the pure Fe$_3$O$_4$ standard (see Table II).

**TABLE II**

Ion titration*

| Iron oxide | FE$^{++}$ (%) | Fe$^{+++}$ (%) | Total Fe |
|---|---|---|---|
| A | 11.3 | 57.0 | 68.3 |
| B | 3.6 | 24.7 | 28.3 |
| Pure Fe$_2$O$_3$ | 2.8 | 67.2 | 70.0 |
| Pure Fe$_3$O$_4$ | 27.6 | 47.7 | 72.3 |

*Method of Kolthoff and Sandell

Thus, these particles have an Fe$^{+++}$ content intermediate to that for pure standards of Fe$_2$O$_3$ and Fe$_3$O$_4$. It is concluded that the excess of iron in the Fe$^{+++}$ valence state is present in non-crystalline form as an amorphous Fe$_2$O$_3$. The Fe$_2$O$_3$ stoichiometry is confirmed by the ESCA and AUGER spectroscopy described hereinabove.

Applicant knows of no technical literature in which such tests have been correlated or conclusions drawn therefrom. As a possible explanation (but without limitation), it may be theorized that each of the present particles consist of a core comprising crystalline Fe$_3$O$_4$, Fe$_2$O$_3$ and combinations thereof, surrounded by an amorphous (non-crystalline) Fe$_2$O$_3$ layer. Reaction with hydrogen sulfide may initiate within the amorphous Fe$_2$O$_3$ layer and proceed into the crystalline core portion. Kinetic analysis, as hereinafter more fully described, indicates a substantially uniphasic reaction, i.e., reaction proceeds uniformly through the core at substantially similar rates to the initial reaction within the amorphous portion, and obeys the same rate law. This suggests that initial reaction energetically primes a subsequent reaction within the crystalline phase. In contrast, in the absence of an amorphous layer, as in mineral magnetite and hematite, reaction proceeds very slowly since the crystal lattice bonding must be overcome before Fe atoms are displaced and made available for chemical reaction.

The proportion of crystalline material (Fe$_2$O$_3$ or Fe$_3$O$_4$) in the present particle does not appear to be critical. The Fe$_3$O$_4$ crystalline portion of compound A is approximately 50% by weight of the total particle; whereas in compound B (open hearth dust) the crystalline phase constitutes only about 9% of the total weight.

In the present process, a liquid slurry of iron oxide particles is contacted with hydrocarbon gas containing hydrogen sulfide. As indicated by the kinetic data hereinafter described, rates of reaction (of fast-reacting compounds A and D) at acidic pH may be 1,000-fold greater than at alkaline pH. However, such a rapid reaction rate is not the only parameter of effectiveness of H$_2$S scavenging. In the aqueous dissociation of dissolved H$_2$S to HS$^-$ and H$^+$, increased acidity increases the partial pressure of H$_2$S in water in accordance with Henry's law. This means that there will be H$_2$S residuals in sweetened hydrocarbon gas, which may actually be greater at lower pH where the rate of reaction is faster than at pH greater than 7. Further, even at pH greater than 7, reaction products of the present iron oxides are acid-stable. Accordingly, in an aqueous suspension of the particles, overall H$_2$S removal may be more efficient at pH greater than 7 even for compounds A and D; and the apparent sacrifice of reaction speed may be compensated by utilizing greater concentrations of the iron oxide.

These factors will be important where hydrocarbon gas flow is relatively rapid and where H$_2$S content is high. Iron oxide concentration, pH and flow rate can readily be adjusted in the present process to achieve the desired H$_2$S residual. By commercial standard, a residual of 4 ppm H$_2$S is tolerated.

Compounds B and C, having a combination of Fe$_2$O$_3$ and Fe$_3$O$_4$ crystalline phases, demonstrate lower reaction efficiency (Table I, kinetic K values) than either compounds A or D whose respective Fe$_3$O$_4$ or Fe$_2$O$_3$ crystalline phases are substantially pure; but both compounds B and C have unusually stable reaction products. Therefore these compounds are adequately useful in the method of the present invention wherein hydrogen sulfide residuals are present in hydrocarbon gases in low concentrations; in such situations, it is important merely that dissolved sulfides be removed as acid insoluble products of reaction, so as to avoid the regeneration of H$_2$S gas.

Reaction kinetics of various iron oxides in reaction with hydrogen sulfide were compared to kinetic results reported in the literature. In the pH range 8—10, the derived rate law for compound A is as follows:

**0 067 812**

$$\frac{d[S_t]}{dt} = -K \times [H^+]^{1.06} \times [A]$$

wherein $[S_t]$ is sulfide concentration in ppm, t is time in minutes, $d[S_t]/dt$ is the instantaneous rate of change of dissolved sulfide concentrations, $[H^+]$ is hydrogen ion concentration and $[A]$ is iron oxide concentration $kg/m^3$. K is the rate constant in $min^{-1}$ $ppm^{-1}$ $cm^{-2} \times$ liters/mole and equal to approximately 1,052. At pH 8—10, the derived rate law agrees closely with Richard's analysis of the reaction of hydrated iron oxide (ferric hydroxide) and hydrogen sulfide [*Am. J. Sci.*, 274:941 (1974)].

Substitution of observed $[S_t]$ and $[H^+]$ values at low pH into the rate law equation hereinabove, yields apparent K values which define relative differences in reaction rates among different compounds. In general, it is found that any iron oxide particle attaining a maximum K value greater than 20 when $[S_t]$ and $[H^+]$ are measured intermittently during the course of continuous acidic reaction, will be satisfactorily effective in the defined process.

The kinetic K values for each of the present ion oxides and control materials are summarized in Table I. Only the compounds of classes A—D have kinetic K values greater than 7.0 and so would be of sufficient practical reactivity with hydrogen sulfide over both an alkaline and acidic pH range. In contrast, all of the prior art iron oxides give maximum K values of approximately 0.4 or less when measured at acidic pH.

In scavenging hydrogen sulfide from hydrocarbon gases, iron oxides have heretofore been known to form chiefly unstable products of reaction such as $Fe_2S_3$ (ferric sulfide) and FeS (ferrous sulfide), together with lesser amounts of stable species such as $FeS_2$ and $S°$. The unstable products rapidly regenerate $H_2S$ upon acidification. Even if the reaction with hydrogen sulfide itself is conducted under alkaline conditions so as to fix the sulfides in solution (as taught by Sperr, U.S. Patent No. 1,734,307), there is substantial danger of $H_2S$ release from the consumed particles upon acidification through later contact with atmospheric $CO_2$.

The iron oxide particles used in the present process produce unexpectedly stable reaction products even in the presence of strong inorganic acids. These products for compound A can be identified as $FeS_2$, $S°$ and $Fe_3S_4$ by X-ray diffraction. Since X-ray diffraction is essentially semi-quantitative, revealing only materials having a defined crystalline structure, a more functional test of reaction product stability was developed.

In this assay, ferrous sulfide (FeS) was prepared by the reaction of $Fe(NH_4)_2(SO_4)_2$ and $Na_2S$ in water; a black FeS precipitate was recovered and washed to remove unreacted sulfides. In water solution (200 mls.), the FeS precipitate (2400 total ppm sulfide) rapidly regenerates $H_2S$ upon addition of strong acid (10 mls. concentrated HCl). The liberated $H_2S$ is bubbled into a $ZnCO_3$ trap and assayed. In the test series, the iron oxides of Table I were introduced individually into the 200 ml. slurry prior to acidification. Strong acid was then added and the amount of $H_2S$ recovered in a zinc carbonate trap measured after one-half hour. The rationale of these experiments is as follows: if the $H_2S$ liberated from the FeS precipitate upon acidification reacts with the iron oxide to produce FeS or other species of iron sulfide unstable in the presence of acid, these unstable reaction products will regenerate sulfide as $H_2S$ gas which bubbles into the zinc carbonate trap. However, if the liberated $H_2S$ reacts with the test iron oxide to produce an acid-stable product of reaction, it will be fixed in the reaction vessel and will not pass into the zinc carbonate trap.

For purpose of this application products of reaction are to be deemed to be acid-stable if they are at least 70% stable in the presence of strong acid when tested by the foregoing assay.

The results of tests are given in Table I and are expressed as the percent of total sulfides derived from FeS which remain fixed in the reaction vessel upon acidification and are not released as $H_2S$ into the zinc carbonate trap. From the data of Table I, each of the classes of compounds A—D, effective in the present process, forms reaction products at least 70% stable in the presence of strong acids. In contrast, all the prior art compounds and control materials yield reaction products only about 10% to 20% stable in the presence of acid. Such reaction products, even if formed under alkaline conditions, pose a future danger of regenerating hydrogen sulfide upon contact with acidic compounds in the environment.

Where very rapid reaction rates are desired, impurity levels of various elements in bulk may significantly affect scavenging efficiency. Even compounds having large surface area (greater than 4 $m^2/g$) and the characteristic $Fe_2O_3$ or $Fe_3O_4$ crystalline phase as hereinabove described, are presently deemed to be unsatisfactory in the present process if any free element of impurity exceeds about 10% by weight. For example, all the control prior art iron oxides designated Q and R of Table I have greater than 10% by weight of at least one such element; all of them have correspondingly low kinetic K values of reaction during the course of continuous flow reaction at acidic pH. In contrast, compounds A—D are found to have low levels of such bulk impurities (less than about 5% by weight) as measured by X-ray fluorescence, these compounds attain a kinetic K value of at least 7.0 at acidic pH during the course of continuous flow reaction.

The surface area of iron oxides is correlated with their chemical reactivity. Highly dense crystalline compounds such as magnetite or hematite have very low surface area, generally less than 1.0 $m^2/g$, and corresponding low reactivity with hydrogen sulfide. Low reactivity thus reflects both the smaller effective surface area available for reaction and the relatively low free energy of these substances due to crystalline lattice bonding. The present iron oxides, useful in the present process, may therefore be characterized or defined by the presence of substantial amorphous iron oxide content, or alternatively by their effective

5

surface area and low density. Reactive compounds must have a surface area of greater than 4 $m^2/g$ and may have a lesser density than that of crystalline hematite or magnetite; they may reveal a substantial amorphous portion together with a crystalline iron oxide phase upon the more elaborate analysis by ESCA/AUGER, X-ray diffraction, and ion titration analysis hereinabove described.

In the method of the present invention, iron oxide particles are added to a liquid to form a slurry or suspension. Hydrocarbon gas is contacted with the slurry so as to react any hydrogen sulfide present with the particles. Any apparatus adapted to bring a liquid phase into intimate contact with the gas phase will be effective in this method, as scrubbing columns, static mixers, bubble vessels, and the like. The sweetened hydrocarbon gas then passes from the liquid slurry and is collected conventionally as it escapes from the slurry.

Contrary to prior art teachings, the slurry of the present process may utilize any liquid capable of practical use with the aforementioned apparatus. In experiments utilizing a Parr pressure bomb apparatus, recovery of sulfur-containing solids was fully as efficient where anhydrous diesel fuel was substituted for water in forming a test slurry of desiccated iron oxide of the type designated compounds A—D, residual water content of desiccated samples was less than 0.2%. These results were not to be expected, in view of Simon and Reichelt [Z. Anerg. Allg. Chem. 319:962 (1964)] which teaches that iron oxide reacts with hydrogen sulfide by being first hydrated to FeOOH; that the hydrogen sulfide becomes ionized to form $HS^-$ and $H^+$; and the reaction proceeds between the FeOOH and the $HS^-$, at an optimum water content of 18.0%.

Industrial applicability:

Benefits of the present invention, as compared with conventional regenerative processes (amine type) include the following: the reactive vessel design is of far greater simplicity and less cost, and may accommodate long intervals between change-out of the reactant material, as when gas wells are remote from servicing personnel. At each change-out the down-time will be substantially less. The reacted material being inert, it is easily disposed of; while the regenerative processes produce concentrated hydrogen sulfide which, if flared off, pollutes the atmosphere with sulfur dioxide; otherwise it must be converted into sulfur.

As compared with the iron sponge process, the present process will remove far greater concentrations of hydrogen sulfide, change-out is faster and more simple; and the reaction products may be simply disposed of; whereas in the iron sponge process unstable forms of iron sulfide are generated.

Definition:

In the claims, the rate contsant K, measured as $min^{-1}$ $ppm^{-1}$ $cm^{-2}\times$liters/mole, is calculated for iron oxide concentrations measured in $kg/m^3$, and is as appears in the following derived rate law:

$$\frac{d[S_t]}{dt} = -K\times[H^+]^{1.06}\times[A]$$

where:

[$S_t$] is sulfide concentration in pppm.

t is time in minutes.

$d[S_t]/dt$ is the instantaneous rate of change of dissolved sulfide concentrations.

[$H^+$] is hydrogen ion concentration.

[A] is iron oxide concentration $kg/m^3$.

## Claims

1. A process of scavenging hydrogen sulfide from hydrocarbon gas, comprising the steps of bringing such hydrocarbon gas into contact with iron oxide particles, characterized in that the iron oxide particles have a crystalline phase portion selected from the group consisting of $Fe_2O_3$, $Fe_3O_4$ and combinations thereof, together with an amorphous $Fe_2O_3$ portion and having a surface area of at least 4 $m^2/g$, and in that the said iron oxide particles are suspended in a liquid slurry, and in that the hydrocarbon gas containing such hydrogen sulfide to be scavenged is bubbled through said slurry, thereby aiding its circulation and thus maintaining said iron oxide particles in suspension, the hydrocarbon gas then escaping from said slurry being collected by known method.

2. A process as defined in claim 1, further characterized in that the said particles have impurity levels of any free element in bulk of less than 10.0% by weight.

3. A process as defined in claim 1, further characterized in that the said particles have an Fe+++ content intermediate to that for pure standards of $Fe_2O_3$ and $Fe_3O_4$.

4. A process as defined in claim 1, further characterized in that said particles comprise a crystalline $Fe_3O_4$ core surrounded by an amorphous $Fe_2O_3$ layer.

5. A process as defined in claim 1, further characterized in that said particles with such hydrocarbon

6

sulfide in a reaction attain a maximum kinetic K value of greater than 20 during the course of continuous acidic reaction.

6. A process as defined in claim 1, further characterized in that said liquid of the slurry is water.

7. A process as defined in claim 1, further characterized in that said liquid of the slurry is a hydrocarbon-based oil.

8. A process as defined in claim 1, further characterized in that said iron oxide particles are basic oxygen furnace dust.

9. A process as defined in claim 1, further characterized in that iron oxide particles are open hearth dust.

**Patentansprüche**

1. Verfahren zum Entfernen von Schwefelwasserstoff aus Kohlenwasserstoffgas, umfassend die Stufen des Kontaktierens des Kohlenwasserstoffgases mit Eisenoxidteilchen, dadurch gekennzeichnet, dass die Eisenoxidteilchen einen kristallinen Phasenanteil ausgewählt aus der Gruppe, bestehend aus $Fe_2O_3$, $Fe_3O_4$ und Kombinationen davon, zusammen mit einem amorphen $Fe_2O_3$-Anteil und mit einer Oberfläche von wenigstens 4 $m^2/g$ haben, und dass die Eisenoxidteilchen in einer flüssigen Aufschlämmung suspendiert sind und wobei das Kohlenwasserstoffgas, von dem der Schwefelwasserstoff entfernt werden soll, durch die Aufschlämmung perlen gelassen wird, wodurch die Zirkulation unterstützt wird und die Eisenoxidteilchen in Suspension gehalten werden, wobei man das aus der Aufschlämmung herauskommende Kohlenwasserstoffgas dann in bekannter Weise sammelt.

2. Verfahren gemäss Anspruch 1, weiterhin dadurch gekennzeichnet, dass die Teilchen ein Verunreinigungsniveau an irgendeinem freien Element in der Masse von weniger als 10,0 Gew.% haben.

3. Verfahren gemäss Anspruch 1, weiterhin dadurch gekennzeichnet, dass die Teilchen einen $Fe^{+++}$ Gehalt haben, der zwischen dem der reinen Standards von $Fe_2O_3$ und $Fe_3O_4$ liegt.

4. Verfahren gemäss Anspruch 1, weiterhin dadurch gekennzeichnet, dass die Teilchen einen kristallinen $Fe_3O_4$-Kern, umgeben von einer amorphen $Fe_2O_3$-Schicht, aufweisen.

5. Verfahren gemäss Anspruch 1, weiterhin dadurch gekennzeichnet, dass die Teilchen mit solchem Schwefelwasserstoff bei einer Reaktion einen maximalen kinetischen K-Wert von mehr als 20 im Laufe der kontinuierlichen sauren Reaktion erhalten.

6. Verfahren gemäss Anspruch 1, weiterhin dadurch gekennzeichnet, dass die Flüssigkeit in der Aufschlämmung Wasser ist.

7. Verfahren gemäss Anspruch 1, weiterhin dadurch gekennzeichnet, dass die Flüssigkeit in der Aufschlämmung ein Öl auf Basis eines Kohlenwasserstoffs ist.

8. Verfahren gemäss Anspruch 1, weiterhin dadurch gekennzeichnet, dass die Eisenoxidteilchen Staub aus einem basischen Sauerstoffofen sind.

9. Verfahren gemäss Anspruch 1, weiterhin dadurch gekennzeichnet, dass die Eisenoxidteilchen Staub aus einem Siemens-Martin-Ofen sind.

**Revendications**

1. Procédé pour éliminer le sulfure d'hydrogène d'un gaz hydrocarboné, comprenant les stades de mise de ce gaz hydrocarboné en contact avec des particules d'oxyde de fer, caractérisé en ce que les particules d'oxyde de fer ont une portion de phase cristalline choisie dans le groupe constitué par $Fe_2O_3$, $Fe_3O_4$ et leurs combinaisons, ensemble avec une portion amorphe de $Fe_2O_3$, et ayant une surface spécifique d'au moins 4$m^2/g$ et en ce que lesdites particules d'oxyde de fer sont en suspension dans une bouillie liquide, et en ce que l'on fait barboter le gaz hydrocaboné contenant ce sulfure d'hydrogène à éliminer à travers ladite bouillie, pour favoriser sa circulation et maintenir ainsi lesidites particules d'oxyde de fer en suspension, le gaz hydrocarboné qui s'échappe alors de ladite bouillie étant récupéré selon un procédé connu.

2. Procédé tel que défini dans la revendication 1, caractérisé de plus en ce que lesdites particules ont des teneurs en impuretés constituées de tout élément libre globalement inférieures à 10,0% en poids.

3. Procédé tel que défini dans la revendication 1, caractérisé de plus en ce que lesdites particules ont une teneur en $Fe^{+++}$ intermédiaire entre celles des normes de pureté pour $Fe_2O_3$ et $Fe_3O_4$.

4. Procédé tel que défini dans la revendication 1, caractérisé de plus en ce que lesdites particules comprennent un noyau cristallin de $Fe_3O_4$ entouré d'une couche amorphe de $Fe_2O_3$.

5. Procédé tel que défini dans la revendication 1, caractérisé de plus en ce que lesdites particules avec ledit sulfure d'hydrogène dans une réaction atteignent une value cinétique K maximale supérieure à 20 au cours de la réaction acide continue.

6. Procédé tel que défini dans la revendication caractérisé de plus en ce que ledit liquide de la bouillie est de l'eau.

7. Procédé tel que défini dans la revendication 1, caractérisé de plus en ce que ledit liquide de la bouillie est une huile à base hydrocarbonée.

8. Procédé tel que défini dans la revendication 1, caractérisé de plus en ce que lesdites particules d'oxyde de fer sont des poussières d'un convertisseur à soufflage d'oxygène au-dessus du bain.

9. Procédé tel que défini dans la revendication 1, caractérisé de plus en ce que les particules d'oxyde de fer sont des poussières de four Martin.